(19) Europäisches Patentamt
European Patent Office
Office européen des brevets

(11) **EP 1 541 096 B1**

(12) **EUROPÄISCHE PATENTSCHRIFT**

(45) Veröffentlichungstag und Bekanntmachung des Hinweises auf die Patenterteilung:
**10.02.2010 Patentblatt 2010/06**

(51) Int Cl.:
***A61F 2/44*** *(2006.01)*

(21) Anmeldenummer: **04024537.5**

(22) Anmeldetag: **14.10.2004**

(54) **Höheneinstellbares Zwischenwirbelimplantat**

Height adjustable intervertebral implant

Implant intervertébral à hauteur réglable

(84) Benannte Vertragsstaaten:
**CH DE FR GB LI**

(30) Priorität: **09.12.2003 DE 10357960**
**09.12.2003 US 528412 P**

(43) Veröffentlichungstag der Anmeldung:
**15.06.2005 Patentblatt 2005/24**

(73) Patentinhaber: **BIEDERMANN MOTECH GmbH**
**78054 VS-Schwenningen (DE)**

(72) Erfinder:
• **Biedermann, Lutz**
**78048 VS-Villingen (DE)**

• **Matthis, Wilfried**
**79367 Weisweil (DE)**

(74) Vertreter: **Hofer, Dorothea et al**
**Prüfer & Partner GbR**
**Patentanwälte**
**Sohnckestrasse 12**
**81479 München (DE)**

(56) Entgegenhaltungen:
**WO-A-01/49220 WO-A-02/080823**
**WO-A-2004/073563 FR-A- 2 817 463**
**US-A- 5 865 848 US-A1- 2002 128 716**

**Beschreibung**

Die vorliegende Erfindung bezieht sich auf ein höheneinstellbares Zwischenwirbelimplantat.

Zwischenwirbelimplantate werden insbesondere nach der Entfernung einer Bandscheibe eingesetzt, um den Zwischenraum zwischen den Wirbelkörpern zu stabilisieren.

Ein solches Zwischenwirbelimplantat ist in der EP 0 977 529 B1 beschrieben. Es ist im Wesentlichen quaderförmig ausgebildet und weist zwei Seitenwände, eine Vorderwand und eine Rückwand auf. Die Boden- und Deckfläche sind offen ausgebildet. In den von den vier Wänden umschlossenen Hohlraum enthält das Zwischenwirbelimplantat wenigstens ein Element mit einer zur Deckfläche bzw. zur Bodenfläche gerichteten Oberfläche und ein in der Vorderwand und in der Rückwand gelagertes Einstellelement, so dass das Element zwischen einer ersten Stellung, in der seine Oberfläche nicht über die Deckfläche bzw. Bodenfläche hervorsteht, und einer zweiten Stellung, in der seine Oberfläche über die Deckfläche bzw. Bodenfläche hervorsteht, hin und her bewegt werden kann. Dadurch kann die Endhöhe des Zwischenwirbelimplantats eingestellt werden.

WO 01/49220 A1 beschreibt ein Zwischenwirbelimplantat, bei dem in einer Ausführungsform ein Hauptteil vorgesehen ist, das mit einem Erweiterungsteil verlängert werden kann. Das Erweiterungsteil weist einen Vorsprung auf, der in einem entsprechend geformten Schlitz des Hauptteils aufgenommen werden kann.

US 5,865,848 beschreibt ein höhenverstellbares Zwischenwirbelimplantat, das zwei Elemente enthält, von denen jedes eine Wirbelkontaktfläche, ein Paar Seitenabschnitte und eine Endplatte aufweist. Die beiden Elemente sind komplementär zueinander. Die Seitenabschnitte sind mit Sägezahnrampen versehen, die bei einer horizontalen Verschiebung wie bei einer Ratsche immer wieder voneinander getrennt werden und wieder ineinander einrasten.

Es ist auch ein einstückig ausgebildetes bananenförmig gekrümmtes Zwischenwirbelimplantat bekannt. Dieses ist jedoch in seiner Höhe nicht verstellbar.

Die Aufgabe der vorliegenden Erfindung besteht darin, ein höhenverstellbares Zwischenwirbelimplantat bereitzustellen, das besonders für einen minimalinvasiven Einsatz geeignet ist.

Die Aufgabe wird gelöst durch ein Zwischenwirbelimplantat gemäß Anspruch 1. Weiterbildungen der Erfindung sind in den Unteransprüchen gekennzeichnet.

Bei diesem Zwischenwirbelimplantat kann durch eine Verschiebung zwischen dem ersten Teil und dem zweiten Teil durch Gleiten der Kontaktflächen aufeinander die Einbauhöhe verändert werden.

Dadurch, dass die Gleitbewegung zwischen dem ersten Teil und dem zweiten Teil vorzugsweise schrauben- bzw. wendelförmig verläuft, ist das Zwischenwirbelimplantat besonders für den minimalinvasiven Einsatz geeignet.

Durch den vorzugsweise vorgesehenen Endanschlag wird eine Gleitbewegung zwischen dem ersten Teil und dem zweiten Teil über eine Endposition hinaus verhindert.

Durch die vorzugsweise vorgesehene Selbsthemmung wird ein unbeabsichtigtes Verschieben zwischen dem ersten Teil und dem zweiten Teil verhindert.

Weitere Merkmale und Zweckmäßigkeiten der Erfindung ergeben sich aus der Beschreibung von Ausführungsbeispielen anhand der beigefügten Zeichnungen.

Von den Figuren zeigen:

Fig. 1    eine perspektivische Ansicht eines Zwischenwirbelimplantats nach einer Ausführungsform der vorliegenden Erfindung in nicht zusammengeschobenem Zustand;

Fig. 2    eine perspektivische Ansicht des in Fig. 1 gezeigten Zwischenwirbelimplantats in zusammengeschobenem Zustand;

Fig. 3    eine perspektivische Ansicht eines ersten Teils des Zwischenwirbelimplantats;

Fig. 4    eine perspektivische Ansicht eines zweiten Teils des Zwischenwirbelimplantats;

Fig. 5    eine Ansicht des Zwischenwirbelimplantats in nicht zusammengeschobenem Zustand;

Fig. 6    eine Ansicht des Zwischenwirbelimplantats in zusammengeschobenem Zustand;

Fig. 7    eine schematische Darstellung einer durch die wirkenden Kräfte bewirkten Selbsthemmung; und

Fig. 8    eine perspektivische Ansicht einer Abwandlung des in Fig. 1 und 2 gezeigten Zwischenwirbelimplantats.

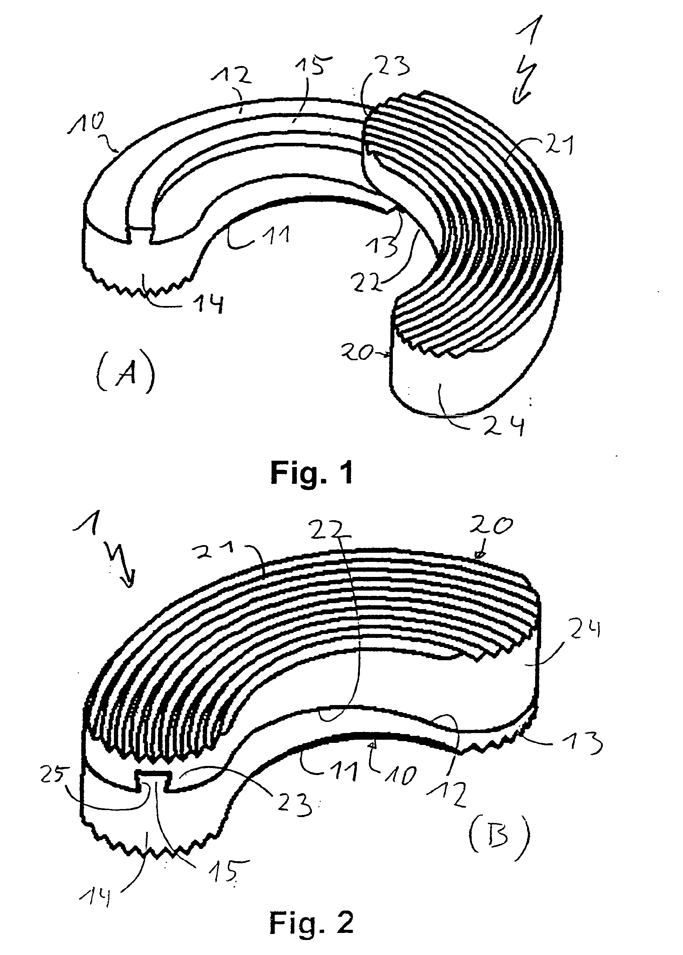

Im folgenden wird mit Bezug auf Fig. 1 bis 6 ein Zwischenwirbelimplantat gemäß einer Ausführungsform der vorliegenden Erfindung beschrieben.

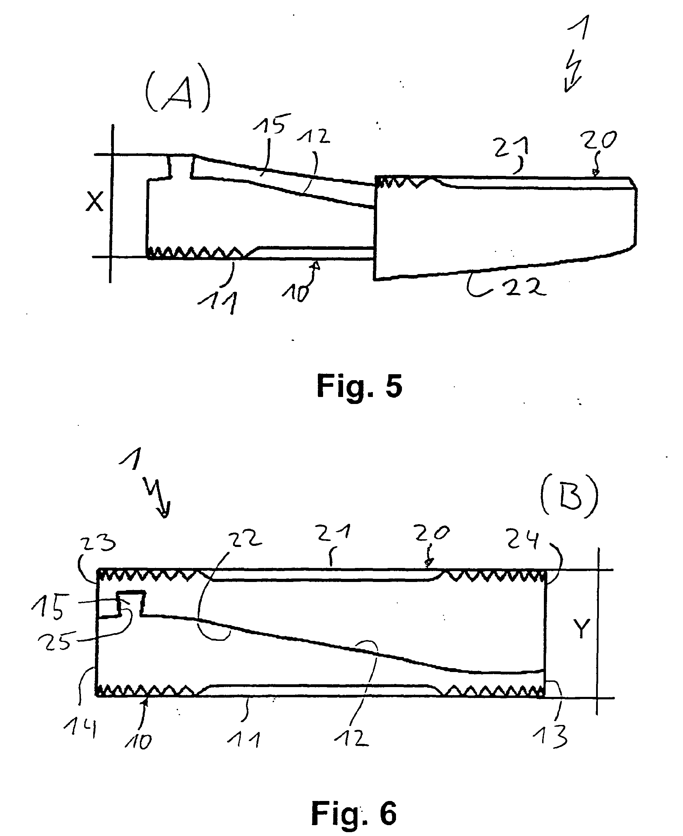

Wie aus Fig. 1 und Fig. 2 ersichtlich, ist das Zwischenwirbelimplantat 1 aus zwei gebogenen Teilen 10, 20 zusammengesetzt, die in der konkreten Ausführungsform kreisbogenförmig ausgebildet sind. Wie am besten aus Fig. 6 ersichtlich, sind die beiden Teile 10, 20 in der Form sich entgegengesetzt verjüngender Keilabschnitte mit jeweils einem den Keilboden bildenden dicken Ende 14, 24 und einem diesem gegenüberliegenden verjüngten Ende 13, 23 ausgebildet. Dabei haben die beiden Teile 10, 20 mit jeweils einer ihrer Keilflächen 12, 22 Kontakt zueinander, weshalb diese Keilflächen im Folgenden auch als Kontaktflächen bezeichnet werden. Das Zwischenwirbelimplantat 1 weist also von der Seite aus gesehen eine annähernd rechteckige Form auf. Durch Verschieben der beiden Teile 10, 20 gegeneinander entlang ihrer Kontaktflächen 12, 22 ist die Gesamthöhe des Zwischenwirbelimplantats 1 einstellbar.

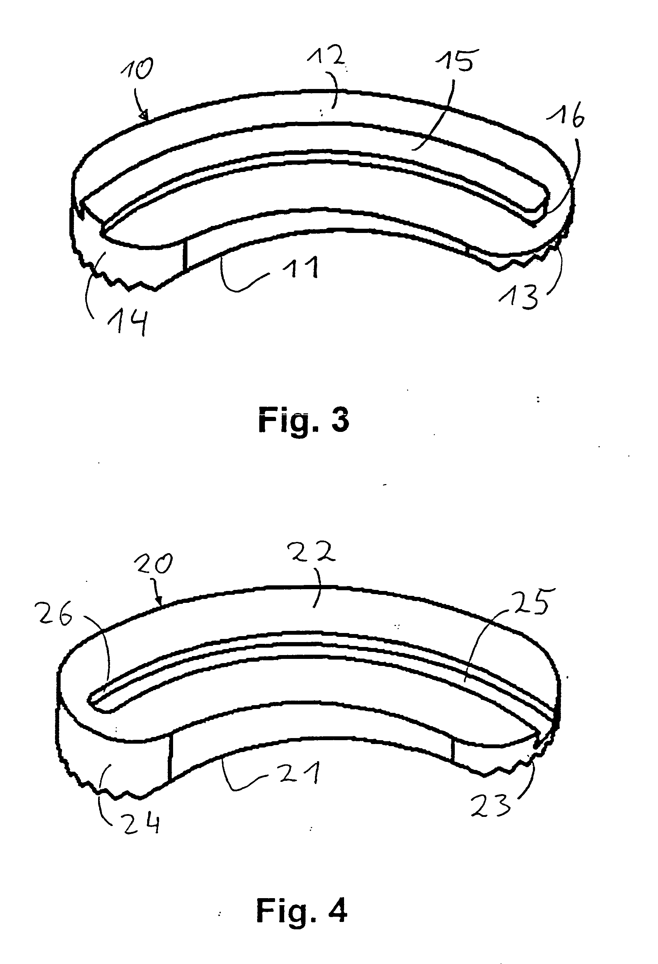

Wie am besten aus Fig. 3 ersichtlich, weist das erste Teil 10 eine erste Kontaktfläche 12 und eine dieser gegenüberliegende erste Oberfläche 11 auf. Die erste Kontaktfläche 12 ist in Form eines Abschnitts einer Schraubenfläche bzw. einer Wendelfläche ausgebildet. Auf der ersten Kontaktfläche 12 ist ein senkrecht aus der Fläche hervorstehender Steg 15 vorgesehen, der einen schwalbenschwanzförmigen Querschnitt aufweist. Der Steg 15 ist schrauben- bzw. wendelförmig ausgebildet. Der Steg 15 erstreckt sich von dem den Keilboden bildenden zweiten Ende 14 des ersten Teils 10 aus bis zu einem vorbestimmten Abstand von dem verjüngten ersten Ende 13 hin.

Wie am besten aus Fig. 4 ersichtlich, weist das zweite Teil 20 eine zweite Kontaktfläche 22 und eine dieser gegenüberliegende zweite Oberfläche 21 auf. Auch die zweite Kontaktfläche 22 ist in Form eines Abschnitts einer Schraubenfläche bzw. einer Wendelfläche ausgebildet, die zu derjenigen der ersten Kontaktfläche 12 komplementär ist. In der zweiten Kontaktfläche 22 ist eine Nut 25 vorgesehen, die einen schwalbenschwanzförmigen Querschnitt aufweist, der dem Querschnitt des Stegs 15 entspricht. Die Nut 25 ist schrauben- bzw. wendelförmig ausgebildet. Die Nut 25 erstreckt sich von dem verjüngten ersten Ende 23 des zweiten Teils 20 aus bis zu einem vorbestimmten Abstand von dem den Keilboden bildenden zweiten Ende 24 hin.

Die erste und zweite Oberfläche (11, 21) sind mit rillenförmigen Vertiefungen versehen, die in der konkreten Ausführungsform in der Draufsicht kreisbogenförmig ausgebildet sind.

Zum Einstellen der gewünschten Höhe des Zwischenwirbelimplantats 1 werden das erste Teil 10 und das zweite Teil 20 entlang ihren Kontaktflächen 12, 22 zwischen einer ersten Stellung A (s. Fig. 1 und 5) und einer zweiten Stellung B (s. Fig. 2 und 6) schraubenförmig gegeneinander verschoben, wobei der Steg 15 in der Nut 25 geführt wird. Die zweite Stellung B ist erreicht, wenn das Stegende 16 gegen das Nutende 26 stößt. Durch das Nutende 26 wird verhindert, dass ein weiteres Verschieben der Teile über die Endposition hinaus erfolgt.

Wie am besten aus Fig. 5 und 6 ersichtlich, weist das Zwischenwirbelimplantat 1 durch die Neigung der Kontaktflächen 12, 22 gegenüber den Oberflächen 11, 21 nach dem Zusammenschieben in die zweite Stellung B eine größere Höhe Y auf als die Höhe X in der ersten Stellung A vor dem Zusammenschieben. Somit ist es möglich, durch eine Gleitbewegung des ersten Teils 10 und des zweiten Teils 20 relativ zueinander die Höhe des Zwischenwirbelimplantats 1 zwischen den beiden Grenzwerten X und Y einzustellen.

Zum Einsetzen des Zwischenwirbelimplantates 1 zwischen zwei Wirbelkörper wird durch Verschieben des ersten Teils 10 und des zweiten Teils 20 gegeneinander die gewünschte Höhe eingestellt und das Zwischenwirbelimplantat 1 zwischen die Wirbelkörper eingesetzt.

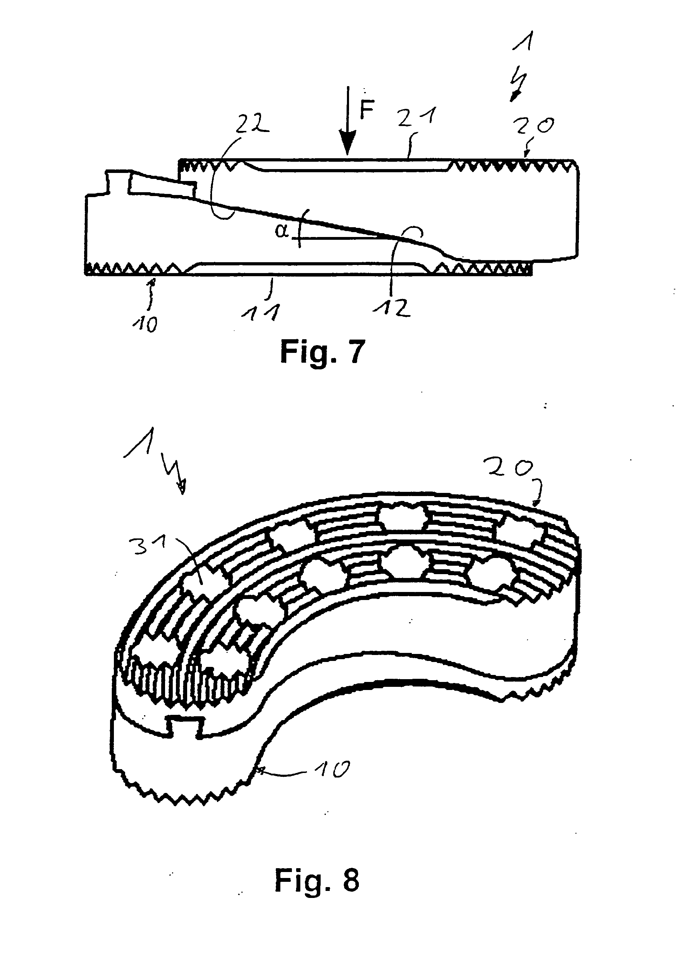

Fig. 7 zeigt eine nach dem Einsetzen zwischen die Wirbelkörper auf das Zwischenwirbelimplantat 1 wirkende Kraft F. Um zu verhindern, dass durch diese Kraft F eine ungewollte Relativbewegung zwischen erstem Teil 10 und zweitem Teil 20 auftritt, ist der Steigungswinkel $\alpha$ der Kontaktflächen 12, 22, unter dem die Schrauben- bzw. Wendellinie gegenüber einer auf der Schraubenachse senkrecht stehenden Ebene (in diesem Ausführungsbeispiel die Oberfläche 11, 21 des jeweiligen Teils 10, 20) ansteigt, zum Erzielen einer Selbsthemmung abhängig von dem verwendeten Material und der Oberflächenbeschaffenheit der Kontaktflächen 12, 22 so gewählt, dass die dafür geltende Bedingung

$$\alpha < \rho_0 \qquad (\text{mit } \rho_0 = \text{arc tan } \mu_0)$$

im Wesentlichen erfüllt ist, wobei $\rho_0$ den Reibungswinkel und $\mu_0$ den Haftreibungskoeffizienten bezeichnet.

Als Material für das Zwischenwirbelimplantat ist vorzugsweise Titan vorgesehen. Alternativ dazu können auch andere körperverträgliche Metalle oder Legierungen oder Kunststoffe wie z.B. PEEK oder PTFE verwendet werden.

Im Folgenden sind Abwandlungen des oben beschriebenen Zwischenwirbelimplantats beschrieben.

In der in Fig. 8 gezeigten Ausführungsform weisen das erste Teil 10 und/oder das zweite Teil 20 in ihren Außenflächen als Bohrungen ausgebildete Ausnehmungen 31 auf, durch die Knochenmaterial in das Zwischenwirbelimplantat 1 einwachsen kann.

In einer weiteren abgewandelten Ausführungsform sind der Steg 15 und die Nut 25 so ausgebildet, dass sie sich jeweils von dem ersten Ende 13, 23 des jeweiligen Teils 10, 20 aus bis zu dessen zweitem Ende 14, 24 hin erstrecken.

Dadurch wird eine Verschiebung zwischen den beiden Teilen über die zweite Stellung B hinaus ermöglicht, so dass der Einstellbereich für die Einbauhöhe vergrößert ist.

In weiteren abgewandelten Ausführungsformen ist die Steg-Nut-Verbindung T-förmig, rechteckig, quadratisch oder in Form einer anderen formschlüssigen Verbindung ausgebildet.

In einer weiteren abgewandelten Ausführungsform ist die Steg-Nut-Verbindung nicht als Abschnitt einer Schrauben- bzw. Wendellinie ausgebildet, sondern gerade.

In einer weiteren abgewandelten Ausführungsform ist die äußere Form des ersten und/oder zweiten Teils nicht kreisbogenförmig ausgebildet. Es reicht, dass die die Steg-Nut-Verbindung als Abschnitt einer Schrauben- bzw. Wendellinie oder gerade ausgebildet ist.

In einer weiteren abgewandelten Ausführungsform sind die Kontaktflächen des ersten und/oder zweiten Teils aufgeraut .

In einer weiteren abgewandelten Ausführungsform sind die Oberflächen des ersten und/oder zweiten Teils ohne Rillen ausgebildet.

**Patentansprüche**

1. Zwischenwirbelimplantat (1) mit
einem ersten Teil (10) und einem zweiten Teil (20) mit aneinander angrenzenden Kontaktflächen (12, 22) und einer Führungseinrichtung (15, 25), die ein stufenfreies Verschieben des ersten Teils (10) und des zweiten Teils (20) relativ zueinander durch Gleiten der Kontaktflächen (12, 22) aufeinander von einer ersten Stellung (A) aus, bei der das erste Ende (13) des ersten Teils (10) dem ersten Ende (23) des zweiten Teils gegenüberliegt, in Richtung auf eine zweite Stellung (B) hin, bei das erste Ende (13) des ersten Teils (10) dem zweiten Ende (24) des zweiten Teils und das zweite Ende (14) des ersten Teils (10) dem ersten Ende (23) des zweiten Teils gegenüberliegt, führt;
**dadurch gekennzeichnet, dass**
der Abstand zwischen der ersten Kontaktfläche (12) des ersten Teils (10) und einer dieser gegenüberliegenden ersten Oberfläche (11) an einem ersten Ende (13) des ersten Teils (10) kleiner ist als an einem zweiten Ende (14), der Abstand zwischen der zweiten Kontaktfläche (22) des zweiten Teils (20) und einer dieser gegenüberliegenden zweiten Oberfläche (21) an einem ersten Ende (23) des zweiten Teils (20) kleiner ist als an einem zweiten Ende (24), und
ein Steigungswinkel ($\alpha$) der Kontaktflächen (12, 22) abhängig von dem verwendeten Material und der Oberflächenbeschaffenheit der beiden Kontaktflächen so ausgebildet ist, dass eine Selbsthemmung zwischen dem ersten Teil (10) und dem zweiten Teil (20) wirksam ist.

2. Zwischenwirbelimplantat (1) nach Anspruch 1, **dadurch gekennzeichnet, dass** die Kontaktflächen (12, 22) und die Führungseinrichtung (15, 25) schrauben- bzw. wendelförmig ausgebildet sind.

3. Zwischenwirbelimplantat (1) nach einem der Ansprüche 1 bis 2, **gekennzeichnet durch** einen Endanschlag (16, 26) für das Aufeinandergleiten der Kontaktflächen (12, 22).

4. Zwischenwirbelimplantat (1) nach einem der Ansprüche 1 bis 3, **dadurch gekennzeichnet, dass** die Führungseinrichtung (15, 25) in Form einer Nut-Steg-Verbindung ausgebildet ist.

5. Zwischenwirbelimplantat (1) nach Anspruch 4, **dadurch gekennzeichnet, dass** die erste Kontaktfläche (12), die zweite Kontaktfläche (22), der Steg (15) und die Nut (25) schrauben- bzw. wendelförmig ausgebildet sind.

6. Zwischenwirbelimplantat (1) nach Anspruch 4 oder 5, **dadurch gekennzeichnet, dass** die Nut (25) in einem vorbestimmten Abstand von dem zweiten Ende (24) der zweiten Kontaktfläche (22) endet, so dass ein Endanschlag (26) für das Aufeinandergleiten der Kontaktflächen (12, 22) gebildet ist.

7. Zwischenwirbelimplantat (1) nach einem der Ansprüche 4 bis 6, **dadurch gekennzeichnet, dass** der Querschnitt des Stegs (15) schwalbenschwanzförmig oder T-förmig oder rechteckig oder quadratisch ausgebildet ist und dass die Nut (25) komplementär dazu ausgebildet ist.

8. Zwischenwirbelimplantat (1) nach einem der Ansprüche 1 bis 7, **dadurch gekennzeichnet, dass** das erste Teil (10) und/oder das zweite Teil (20) Öffnungen aufweisen.

9. Zwischenwirbelimplantat (1) nach einem der Ansprüche 1 bis 8, **dadurch gekennzeichnet, dass** die erste Kontaktfläche (12) und/oder die zweite Kontaktfläche (22) aufgeraut ausgebildet sind.

10. Zwischenwirbelimplantat (1) nach einem der Ansprüche 1 bis 9, **dadurch gekennzeichnet, dass** die erste Oberfläche (11) und/oder die zweite Oberfläche (21) rillenförmige Vertiefungen aufweisen.

**Claims**

1. Intervertebral implant (1), with
a first part (10) and a second part (20) having mutually adjoining contact faces (12, 22), and a guide mechanism (15, 25) which guides a stepless displacement of the first part (10) and of the second part (20) relative to each other, by sliding of the contact faces (12, 22) on each other, from a first position (A), in which the first end (13) of the first part (10) lies opposite the first end (23) of the second part, towards a second position (B), in which the first end (13) of the first part (10) lies opposite the second end (24) of the second part and the second end (14) of the first part (10) lies opposite the first end (23) of the second part,
**characterized in that**
the distance between the first contact face (12) of the first part (10) and an opposite first surface (11) at a first end (13) of the first part (10) is smaller than at a second end (14),
the distance between the second contact face (22) of the second part (20) and an opposite second surface (21) at a first end (23) of the second part (20) is smaller than at a second end (24), and
a gradient angle ($\alpha$) of the contact faces (12, 22) is designed, depending on the material used and on the surface properties of the two contact faces, such that self-locking is effective between the first part (10) and the second part (20).

2. Intervertebral implant (1) according to Claim 1, **characterized in that** the contact faces (12, 22) and the guide mechanism (15, 25) are screw-shaped or coil-shaped.

3. Intervertebral implant (1) according to one of Claims 1 and 2, **characterized by** an end stop (16, 26) for the sliding of the contact faces (12, 22) on each other.

4. Intervertebral implant (1) according to one of Claims 1 to 3, **characterized in that** the guide mechanism (15, 25) is designed in the form of a mortise and tenon connection.

5. Intervertebral implant (1) according to Claim 4, **characterized in that** the first contact face (12), the second contact face (22), the tenon (15) and the mortise (25) are screw-shaped or coil-shaped.

6. Intervertebral implant (1) according to Claim 4 or 5, **characterized in that** the mortise (25) ends at a predetermined distance from the second end (24) of the second contact face (22), such that an end stop (26) for the sliding of the contact faces (12, 22) on each other is formed.

7. Intervertebral implant (1) according to one of Claims 4 to 6, **characterized in that** the cross section of the tenon (15) is dovetail-shaped or T-shaped or rectangular or square, and **in that** the mortise (25) is shaped to match it.

8. Intervertebral implant (1) according to one of Claims 1 to 7, **characterized in that** the first part (10) and/or the second part (20) have openings.

9. Intervertebral implant (1) according to one of Claims 1 to 8, **characterized in that** the first contact face (12) and/or the second contact face (22) are roughened.

10. Intervertebral implant (1) according to one of Claims 1 to 9, **characterized in that** the first surface (11) and/or the second surface (21) have groove-shaped depressions.

**Revendications**

1. Implant intervertébral (1), avec :

une première partie (10) et une deuxième partie (20) avec des surfaces de contact (12, 22) limitrophes les unes des autres,
un dispositif de guidage (15, 25), guidant un coulissement continu de la première partie (10) et de la deuxième partie (20) l'une par rapport à l'autre, par glissement des surfaces de contact (12, 22) les unes sur les autres, d'une première position (A), pour laquelle la première extrémité (13) de la première partie (10) se trouve en regard de la première extrémité (23) de la deuxième partie, en direction d'une deuxième position (B), pour laquelle la première extrémité (13) de la première partie (10) se trouve en regard de la deuxième extrémité (24) de la deuxième partie, et la deuxième extrémité (14) de la premier partie (10) se trouve en regard de la première extrémité (23) de la deuxième partie ;

**caractérisé en ce que**
l'espacement entre la première surface de contact (12) de la première partie (10) et une première surface (11), opposée à celle-ci, sur une première extrémité (13) de la première partie (10) est inférieur à ce qu'il est à une deuxième extrémité (14),
l'espacement entre la deuxième surface de contact (22) de la deuxième partie (20) et une deuxième surface (21), opposée à celle-ci, sur une première extrémité (23) de la deuxième partie (20) est inférieur à ce qu'il est à une deuxième extrémité (24), et
un angle de pente (α) des surfaces de contact (12, 22), en fonction du matériau utilisé et de l'état de surface des deux surfaces de contact, est réalisé de manière qu'un blocage automatique agisse entre la première partie (10) et la deuxième partie (20).

2.  Implant intervertébral (1) selon la revendication 1, **caractérisé en ce que** les surfaces de contact (12, 22) et le dispositif de guidage (15, 25) sont réalisés en forme de vis ou de spirale.

3.  Implant intervertébral (1) selon l'une des revendications 1 à 2, **caractérisé par** une butée finale (16, 26) pour le glissement l'une sur l'autre des surfaces de contact (12, 22).

4.  Implant intervertébral (1) selon l'une des revendications 1 à 3, **caractérisé en ce que** le dispositif de guidage (15, 25) est réalisé sous la forme d'une liaison à rainure et languette.

5.  Implant intervertébral (1) selon la revendication 4, **caractérisé en ce que** la première surface de contact (12), la deuxième surface de contact (22), la languette (15) et la rainure (25) sont réalisées en forme de vis ou de spirale.

6.  Implant intervertébral (1) selon la revendication 4 ou 5, **caractérisé en ce que** la rainure (25) s'achève à une distance prédéterminée de la deuxième extrémité (24) de la deuxième surface de contact (22), de manière qu'une butée finale (26) soit formée pour le glissement l'une sur l'autre des surfaces de contact (12, 22).

7.  Implant intervertébral (1) selon l'une des revendications 4 à 6, **caractérisé en ce que** la section transversale de la languette (15) est réalisée en forme de queue d'aronde ou en forme de T, ou rectangulaire ou carrée, et **en ce que** la rainure (25) lui est complémentaire.

8.  Implant intervertébral (1) selon l'une des revendications 1 à 7, **caractérisé en ce que** la première partie (10) et/ou la deuxième partie (20) présentent des ouvertures.

9.  Implant intervertébral (1) selon l'une des revendications 1 à 8, **caractérisé en ce que** la première surface de contact (12) et/ou la deuxième surface de contact (22) sont rendues rugueuses.

10. Implant intervertébral (1) selon l'une des revendications 1 à 9, **caractérisé en ce que** la première surface (11) et/ou la deuxième surface (21) présentent des creusements en forme de cannelures.

**Fig. 1**

**Fig. 2**

EP 1 541 096 B1

Fig. 3

Fig. 4

8

**Fig. 5**

**Fig. 6**

**Fig. 7**

**Fig. 8**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0977529 B1
- WO 0149220 A1
- US 5865848 A